# EUROPÄISCHE PATENTSCHRIFT

(11) **EP 3 180 390 B1**
(45) Veröffentlichungstag und Bekanntmachung des Hinweises auf die Patenterteilung: **25.11.2020**
(21) Anmeldenummer: 15744854.9
(22) Anmeldetag: 16.06.2015
(51) Int. Cl.: C08G 79/02, A61L 27/52, A61F 2/28, C08L 85/02

(54) **POLYMER FÜR DIE GEWEBEZÜCHTUNG**
POLYMER FOR TISSUE ENGINEERING
POLYMÈRE POUR LA CULTURE DE TISSUS

(30) Priorität: 16.06.2014 AT 504162014
(43) Veröffentlichungstag der Anmeldung: 21.06.2017
(73) Patentinhaber: Universität Linz, 4040 Linz (AT); Biomed-Zet Life Science GmbH, Linz 4020 (AT)
(72) Erfinder: TEASDALE, Ian, A-4040 Linz (AT); WILFERT, Sandra, 95111 Rehau (DE); AIGNER, Tamara, A-4060 Leonding (AT); ITURMENDI, Aitziber, A-4020 Linz (AT); BRUEGGEMANN, Oliver, A-4073 Wilhering (AT); SCHROEDER, Klaus, A-4040 Linz (AT); OLAWALE, Gbenga, A-4020 Linz (AT); HILDNER, Florian, A-4291 Lasberg (AT); RIGAU DE LLOBET, Maria, 17003 Girona (ES)
(74) Vertreter: Burgstaller, Peter
(86) Internationale Anmeldenummer: PCT/AT2015/050151
(87) Internationale Veröffentlichungsnummer: WO 2015/192158

(56) Entgegenhaltungen:
- EP-A2- 2 540 764
- WO-A1-2008/153278
- GB-A- 2 449 264
- US-A1- 2010 297 155
- US-B1- 6 235 061
- JUNG-KYO CHO ET AL: "Synthesis and characterization of biodegradable thermosensitive neutral and acidic poly(organophosphazene) gels bearing carboxylic acid group", JOURNAL OF POLYMER RESEARCH, KLUWER ACADEMIC PUBLISHERS-CONSULTANTS BUREAU, NL, Bd. 18, Nr. 4, 6. Juli 2010 (2010-07-06), Seiten 701-713, XP019915420, ISSN: 1572-8935, DOI: 10.1007/S10965-010-9466-5

## Beschreibung

Die Erfindung bezieht sich auf ein quervernetztes Polymer für die Gewebezüchtung basierend auf einem bioabbaubaren Polyphosphazen mit photopolymerisierten Seitengruppen sowie auf ein dreidimensionales Gerüst für die Gewebezüchtung unter Einsatz dieses Polymers.

Fortschrittliche Materialien für biomedizinische Anwendungen, beispielsweise für Implantate, für Liefersysteme zur kontrollierten Freisetzung von Wirkstoffen oder für Gerüste zur Gewebezüchtung, basieren auf biologisch abbaubaren Polymeren. Für Polymere, die erfolgreich als Matrizen für die Gewebezüchtung verwendet werden können, werden bestimmte biologische und physikalische Eigenschaften, wie Biokompatibilität, spezifische mechanische Eigenschaften, anpassbare Abbauraten und nicht toxische Abbauprodukte, sowie eine Morphologie gefordert, die das Gewebewachstum unterstützt. Für dieses Anwendungsgebiet sind Poly(organo)phosphazene aufgrund ihrer sehr unterschiedlichen, von den Substituenten der Seitengruppen abhängigen Eigenschaften, vor allem aber der Möglichkeit, die Abbaugeschwindigkeit des anorganischen Rückgrats zu ändern, von besonderem Interesse.

Außerdem bilden die Abbauprodukte zum Unterschied zu sauren Abbauprodukten von vielen anderen in der Biomedizin eingesetzten abbaubaren Polymeren eine nahezu neutrale, pH-gepufferte Mischung aus Phosphaten und Ammoniak, sodass unerwünschte Nebenwirkungen, wie Gewebereizungen und entzündliche oder allergische Reaktionen, weitgehend ausbleiben.

Um die Abbaurate von Polyphosphazenen einstellen zu können, ist es bekannt (US 6 077 916), einerseits hydrophobe Seitengruppen, wie p-Methylphenoxy und andere aromatische Gruppen, und anderseits hydrolytische Seitengruppen, wie Aminosäurealkylester, vorzusehen. So wird beispielsweise die Hydrophobie eines mit Ethylglycinat substituierten Polyphosphazens durch Hinzufügen von p-Methylphenoxy als Co-Substituent erhöht, während die Ethylglycinat-Seitengruppe die biologische Abbaumöglichkeit zu unschädlichen Abbauprodukten in einer wässrigen Umgebung sicherstellt.

Nachteilig ist allerdings, dass die für einen Einsatz auf dem Gebiet der Gewebezüchtung erforderlichen mechanischen Eigenschaften nur mit einem zusätzlichen, nicht auf der Basis eines Polyphosphazens aufgebauten Polymers erreicht werden können.

Zur Bereitstellung hochporöser, dreidimensionaler, biologisch abbaubarer Polymerstrukturen auf der Basis von Polyphosphazenen für die Züchtung von Skelettgeweben ist es außerdem bekannt (US 6 235 061), das Polyphosphazen mit hydrolytisch instabilen Seitengruppen, wie Glucosyl-, Glycinyl-, Glyceryl-, Imidazolyl- oder Ethoxy-Gruppen zu substituieren. Die hohe Porosität wird durch Salze, vorzugsweise Natriumsalze, ermöglicht, die dem in einem organischen Lösungsmittel, bevorzugt Tetrahydrofuran (THF), gelösten Polymer zugemischt werden, um nach einem Verdampfen des Lösungsmittels wieder aus dem ausgehärteten Polymer gelöst zu werden, sodass eine offenporige Polymermatrix mit gleichmäßig über das Volumen verteilten Poren erhalten wird. Nachteilig ist allerdings, dass die mechanischen Eigenschaften dieser bekannten Polymerstrukturen den höheren Anforderungen beim Einsatz als Implantat nicht genügen können.

Die WO 2008153278 A1 zeigt Hydrogele auf Polyphosphazenbasis, wobei das Polyphosphazen vernetzbare Seitengruppen aufweist. Vorteilhaft ist die durch die Quervernetzung erzielbare etwas höhere mechanische Stabilität des Hydrogels. Nachteilig am beschriebenen Polymer ist, dass das Polyphosphazen mehrere unterschiedliche Seitengruppen aufweist, was ein komplexes Herstellungsverfahren erforderlich macht. Nachteilig ist zudem, dass aufgrund der unterschiedlichen Seitengruppen keine ausreichend hohe Quervernetzung erreichbar ist und aufgrund der Verwendung einer PEG-Seitengruppe ein weiches hydrophiles Polymer also ein in Wasser quellendes Hydrogel resultiert. Die EP2540764A2 und die US2010297155A1 betreffen ebenfalls Hydrogele.

Der Erfindung liegt somit die Aufgabe zugrunde, ein Polymer für Gewebezüchtungen der eingangs geschilderten Art so auszugestalten, dass nicht nur vorteilhafte Voraussetzungen für einen einstellbaren, biologischen Abbau geschaffen werden, sondern auch die Voraussetzungen geschaffen werden, mit Hilfe dieser Polymerstrukturen in einfacher Weise für die Gewebezüchtung gut geeignete, dreidimensionale Gerüste zu schaffen, die auch höheren mechanischen Anforderungen entsprechen können.

Zum Lösen der Aufgabe wird ein Polymer für die Gewebezüchtung aus bioabbaubaren Polyphosphazenen mit photopolymerisierten Seitengruppen vorgeschlagen, bei welchem die Seitengruppen der Polyphosphazene ausschließlich aus Aminosäuren und/oder Aminosäurederivaten gebildet sind, welche über die Aminogruppe der Aminosäure am Rückgrat des Polyphosphazens gebunden sind und an der Säuregruppe eine über einen Abstandhalter gebundene Vinylgruppe, also eine reaktive Kohlenstoff-Kohlenstoff-Doppelbindung, aufweisen, wobei für die Länge m der Kohlenstoffkette des Abstandhalters gilt m = 0 bis m = 10 und wobei die Polyphosphazene über die Vinylgruppen der Seitengruppen vernetzt sind.

Beim gegenständlichen Polyphosphazen handelt es sich nicht um ein Hydrogel, wie zur WO 2008153278 A1 erläutert wurde, da dieses unterschiedliche bzw. weitere Seitengruppen voraussetzt und ein Hydrogel per Definition als weiches hydrophiles in Wasser quellendes Polymer nicht zur Lösung der erfindungsgemäßen Aufgabe geeignet ist.

Besonders vorteilhaft ist, dass jede Seitengruppe am Ende eine reaktive Kohlenstoff-Doppelbindung aufweist, wodurch ein verbessert quervernetztes Polymer resultiert. Durch die Quervernetzung lassen sich so starre formstabile Gerüste für die Gewebszüchtung herstellen, welche eine hohe Porosität aufweisen können. Besonders vorteilhaft ist zudem, dass alle Seitengruppen über Peptidbindung der Aminogruppe der Aminosäuren bzw. der Aminosäurederivate am Polyphosphazen-rückgrat binden, wodurch aufgrund der einheitlichen Reaktivität bei Bindung der Seitengruppen der Herstellungsprozess leicht beherrschbar ist, insbesondere auch bei Verwendung unterschiedlicher Aminosäure- bzw. Aminosäurederivat-Seitengruppen das Mengenverhältnis dieser.

Bevorzugt weist das erfindungsgemäße quervernetzbare Polyphosphazen eine Struktur gemäß der Strukturformel 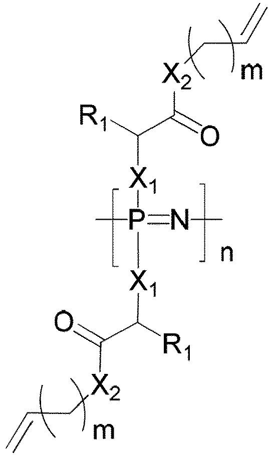
auf, wobei X₁ für NH und X₂ für O, S oder NH steht,
R₁ die Seitenkette von Alanyl, Valinyl, Leucinyl, Isoleucinyl, Prolinyl, Phenylalaninyl, Tryptophanyl, Methioninyl, Glycinyl, Serinyl, Threoninyl, Cysteinyl, Tyrosinyl, Asparaginyl, Glutainyl, Aspartoyl, Glutaoyl, Lysinyl, Argininyl oder Histidinyl ist und wobei gilt m = 0 bis 10 und n = 3 bis 1000.

Die Seitengruppe des Polyphosphazens ist bevorzugt ein Aminosäureester, besonders bevorzugt ein Aminosäurevinylester oder ein Aminosäureallylester. Demgemäß wird die Vinylgruppe bzw. deren Abstandhalter über eine Esterbindung an der Hydroxygruppe der Aminosäure gebunden (X₂ steht für O).

Bei Verwendung eines Aminosäurederivats kann anstelle der Esterbindung eine Amid- (X₂ steht für NH) oder Thioesterbindung (X₂ steht für S) vorliegen.

Ein erfindungsgemäßes Polyphosphazen kann Seitengruppen aus verschiedenen Aminosäureestern bzw. Aminosäurederivaten aufweisen, beispielsweise valine allylester und glycine allylester, bevorzugt weist das erfindungsgemäße Polyphosphazen einheitliche Seitengruppen auf, beispielsweise ausschließlich glycine allylester.
Es ist auch denkbar, die Amidbindung X₁ durch eine Ester- oder Thioesterbindung (X₁ würde für O oder S stehen) zu ersetzen, dies würde aber aus derzeitiger Sicht zu einem Polymer mit eher nachteiligen Eigenschaften für die bevorzugte Anwendung als Gerüst zur Gewebezüchtung führen.

Aufgrund der Seitengruppen kann das vergleichsweise kurzkettige Polyphosphazen einer Photopolymerisation mit dem Ergebnis einer ausgeprägten Quervernetzung mit kovalenten Bindungen unterworfen werden, sodass alle Voraussetzungen zum Herstellen eines dreidimensionalen, quervernetzten Polymergerüsts erfüllt sind, das die mechanischen Eigenschaften für ein als Implantat einsetzbares Gerüst zur Gewebezüchtung mit sich bringt, biokompatibel ist und biologisch abgebaut werden kann.

Der Einbau von zum Rückgrat benachbarten Aminosäure-Spacern wie vorzugsweise Glycin-Spacern bedingt insbesondere eine Beschleunigung der hydrolytischen Abbaubarkeit des Polyphosphazenrückgrats, wobei die reaktive Kohlenstoff-Kohlenstoff-Doppelbindung der Vinylgruppe am Ende jeder Seitengruppe vorteilhafte Voraussetzungen für die anschließende photochemische Vernetzung und Funktionalisierung des Polymers bieten.

Dreidimensionale Gerüste für die Gewebezüchtung lassen sich auf der Basis von mit Aminosäurevinylestern oder Aminosäureallylestern substituierten Polyphosphazenen besonders vorteilhaft herstellen, wenn das Polyphosphazen photopolymerisiert bzw. photovernetzt wird.

Für die Photoreaktion kann vorteilhaft eine Thiolverbindung, welche zumindest zwei Thiolgruppen aufweist, eingesetzt werden, wobei der Einsatz einer entsprechenden Auswahl an multifunktionalen Thiolen mit unterschiedlichen Abstandhaltern zwischen den zumindest zwei Thiolgruppen die Anpassung der jeweiligen Eigenschaften an die vorgegebenen Anforderungen und die Steuerung des hydrophilen und hydrophoben Verhaltens des fertigen Gerüsts erlaubt. Besonders vorteilhafte Verhältnisse konnten in diesem Zusammenhang sichergestellt werden, wenn als Thiolverbindung ein Thiol-trimethylolpropan- tris(3-mercaptopropionat) eingesetzt wird, welche drei Thiolgruppen aufweist. Die Funktionalisierung über eine Thiol-En-Addition ermöglicht die kovalente Bindung von verschiedenen Molekülen mit einer Thiolgruppe. So kann vorzugsweise die biologische Abbaubarkeit des Polymergerüsts durch die Polymerisation des Polyphosphazens mit einer Thiolverbindung und einem Adipinsäuredivinylester aufgrund der Hydrophobie dieses Esters nachhaltig beeinflusst werden. Die Eigenschaften des dreidimensionalen, quervernetzen Gerüsts können somit einfach eingestellt werden, wobei nur die Synthese eines einzigen Poly(organo)phosphazens mit einheitlichen Seitengruppen erforderlich ist.

Die erforderliche Porosität des dreidimensionalen Gerüsts zur Gewebezüchtung kann auf unterschiedlichen Wegen in an sich bekannter Weise, z. B. durch Stereolithographie oder Schäumen durch ein Treibgas, erreicht werden. Besonders einfache Herstellungsbedingungen ergeben sich allerdings, wenn den Vernetzungskomponenten ein Porogen, vorzugsweise ein Salz, beigemischt wird, das nach der Polymerisierung wieder aus dem Polymerisat ausgelaugt wird und ein zusammenhängendes Porensystem zurücklässt.

Die Erfindung wird an Hand eines Herstellungsbeispiels und einer Zeichnung veranschaulicht:

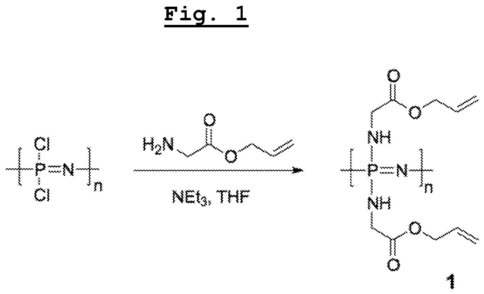

- Fig. 1:: Zeigt das Reaktionsschema zur Herstellung eines beispielhaften erfindungsgemäßen Polyphosphazens mit polymerisierbaren Seitengruppen.

Zur Herstellung eines erfindungsgemäßen Polyphosphazens wurde eine makromolekulare Substitution von Polydichlorphosphazen durchgeführt, das über eine lebende Polymerisation von Trichlorphosphoranimin synthetisiert wurde. Im Handschuhkasten wurden 24,5 mg PCl₅ (0,12 mmol, 1 eq.) und 0,66 g Cl₃P = N-SiMe₃ (2,94 mmol, 25 eq.) in 10 ml wasserfreiem CH₂Cl₂ gelöst und 16 Stunden bei Raumtemperatur gerührt. Das Lösungsmittel wurde unter vermindertem Druck entfernt und das erhaltene Polydichlorphosphazen ohne weitere Reinigung verwendet.

Ausbeute quantitativ: ³¹P-NMR (CDCl3): δ = -18,16 ppm.

Die makromolekulare Substitution von Polydichlorphosphazen erfolgte gemäß dem in Fig. 1 skizzierten Verfahren, wobei das erhaltene Polymer 1 Seitengruppen von Glycin-Allylester aufwies.

Es wurde zunächst 1,52 g 2-(Tert-butoxycarbonylamino)-acetat (7,06 mmol, 2,4 eq.) in Trifluorethansäure(TFA)/CH₂Cl₂ (1/3) 6 Stunden lang entschützt. Die Lösungsmittel wurden sorgfältig unter Vakuum entfernt, um Allyl-2-aminoacetat zu erhalten. Allyl- 2-aminoacetat wurde in wasserfreiem THF gelöst und ein großer Überschuss an NEt3 hinzugefügt, um TFA-Reste zu neutralisieren. In wasserfreiem THF gelöstes Polydichlorphosphazen (0,66 g, 2,94 mmol, 1 eq.) wurde dann der Lösung von Allyl- 2-aminoacetat hinzugefügt. Die Umsetzung wurde 24 Stunden bei Raumtemperatur gerührt. Ausgefälltes Salz wurde durch Filtration entfernt und das Reaktionsgemisch unter Vakuum konzentriert. Das Polymer wurde durch ein Ausfällen aus THF in gekühltem Diethylether gereinigt. Das Polymer wurde dann in Ethylacetat gelöst und weiter mit H₂O und einer Lake gewaschen sowie über MgSO₄ getrocknet. Das Lösungsmittel wurde unter Vakuum entfernt und das Produkt unter Hochvakuum weiter getrocknet, um das Polymer 1 als gelbliches hochviskoses Produkt zu erhalten.

Ausbeute; 0,66 g (80 %) . ¹H-NMR (CDCl₃) : δ = 3,75 (br , 2H), 4,55 (br , 2H), 5,19 bis 5,31 (br, m, 2H), 5,84 bis 5,93 (br, m, 1H) ppm. ³¹P-NMR (CDCl₃) : δ = 1,73 ppm. ¹³C-NMR (CDCl₃) : δ = 42,8 (NH-CH₂), 65,5 (OCH₂), 118,4 (-CH₂), 132,2 (-CH-), 172,5 (C-O) ppm. FTIR (fest): νₘₐₓ = 3341 (NH), 2938 (CH), 1737 (C=O), 1650 (C=C), 1188 (P=N) cm⁻¹.

Um ein poröses, dreidimensionales Gerüst auf Glycinbasis zu erhalten, wurde das Polymer 1 durch Thiol-En-Photopolymerisation vernetzt, und zwar mit Hilfe von Thiol- trimethylolpropan-tris(3-mercaptopropionat) (in Folge als Trithiol bezeichnet). Die Photopolymerisation der Allylgruppen des Polymers 1 und des Trithiols wurde bei Raumtemperatur in Gegenwart eines Porogens in CHCl₃ mit 2,2-Dimethoxy-2-phenylacetophenon (DMPA) als Photoinitiator in kleinen Mengen (ca. 1 Gew.%) durchgeführt. In einem Glasfläschchen wurden das Polymer 1 (90,0 mg , 0,33 mmol, 1 eq.) und 1 mg in 1 ml CHCl₃ gelöst. Dann wurden 0,5 ml Polyethylenglycol mit einem Nennmolekulargewicht von 200 g/mol (PEG-200), Trithiol (72 µl, 87,6 mg, 0,22 mmol, 0,67 eq.) und NaCl als Porogen (ca. 4,2 g, 75 Gew. % der Reaktionsmischung) zugegeben, um eine homogene Mischung mit vollständig dispergierten NaCl-Partikeln zu erhalten.

Das Gemisch wurde in einem UV-Reaktor mit UV-Licht für 1,5 Stunden belichtet.

Das Material wurde aus dem Fläschchen entfernt und zum Auswaschen des Salzes und des PEG-200 wiederholt in einen H₂O-Überschuss gelegt. Die Gerüste wurden durch Soxhlet-Extraktion unter Verwendung von EtOH für 16 Stunden gereinigt und unter Vakuum getrocknet, um ein Polymer 2 als ein poröses Pellet zu erhalten. Die Verfestigung des Reaktionsgemisches zeigte eine erfolgreiche Bildung des quervernetzten Polymernetzwerks um das Porogen.

Ergebnis: ³¹P-NMR (fest): δ = 7,7 ppm. ¹³C-NMR (fest): δ = 7,6 (CH₃), 26,8 (CH₂), 43,8 (NH-CH₂), 65,1 (OCH₂), 172,1 (C-O) ppm. FTIR (fest): νₘₐₓ = 3342 (NH), 2926 (CH), 1729 (C=O), 1188 (P=N) cm⁻¹. Elementaranalyse: berechnet, C 44,57 %, H 6,23%, N 7,80 %, S 11,90 %, P 5,75 %, gefunden, C 43,97 %, H 6,21%, N 7,08 %, S 11,23 % P 5,47 %.

Das Polymer 1 wurde außerdem mit einem kommerziell erhältlichen Adipinsäuredivinylester (VE) in verschiedenen Verhältnissen gemischt, um die Abbaurate der erhaltenen Gerüste zu verändern. Die Bedingungen für die Thiol-En-Vernetzungsreaktion waren bei einer Anpassung des Molverhältnisses von den Alken-Gruppen zu den Thiolgruppen (1/1) ähnlich zu den Thiol-En-Vernetzungsreaktion des Polymers 1.

Für ein Polymer 3 wurden 27 Gew.% des Polymers 1 mit 53 Gew.% Trithiol und 20 Gew.% VE gemischt und einer Thiol-En-Vernetzungsreaktion unterworfen.

Ergebnis: FTIR (fest): νₘₐₓ = 3353 (NH), 2930 (CH), 1728 (C=O), 1184 (P=N) cm-1.

Analyse: berechnet, C 47,91 %, H 6,50 %, N 4,19 %, S 12,79 %, P 3,09 %, gefunden, C 47,50 %, H 6,54 %, N 4,17 %, S 12,36 %, P 3,25 %.

Die Abbauuntersuchungen wurden in entionisiertem H₂O bei 37 °C über 12 Wochen durchgeführt. Proben, die 30 mg der Polymere 2 und 3 enthielten, wurden in versiegelte Fläschchen gegeben und in 2 ml H₂O inkubiert. Eine Datenanalyse wurde jeweils dreifach in entsprechenden Zeitabständen über die Untersuchungsdauer vorgenommen.

Die Proben wurden in einem Vakuumofen bei 40 °C getrocknet, bis das Gewicht konstant war. Der Massenverlust wurde gravimetrisch bestimmt, wobei der jeweils festgestellte Durchschnittswert der Massenverluste als Prozentsatz im Vergleich zum Anfangsgewicht der Abbauprobe in der nachstehenden Tabelle angegeben wird.

| | Gewichtsverlust [%] nach | | | | | |
|---|---|---|---|---|---|---|
| | 10 Tagen | 20 Tagen | 42 Tagen | 56 Tagen | 90 Tagen | 120 Tagen |
| Polymer 2 | 5 | 16 | 33 | 60 | 62 | 85 |
| Polymer 3 | 0 | 0 | 7 | 7 | 20 | 26 |

Die Tabelle zeigt, dass das Polymer 2, das den höchsten Anteil an Polyphosphazenen aufwies, ein ausgeprägtes Abbauprofil in einer neutralen wässrigen Lösung bei 37 °C zeigte. Als Folge des hydrolytischen Abbaus des Polyphosphazenrückgrats werden die Netzwerkbindungen der vernetzten organischen Masse getrennt, was zu einer Verbesserung der Gesamtabbaurate des Gerüsts führt. Mit der Abnahme des Polyphosphazenanteils und der Zunahme des Adipinsäuredivinylesters wird die Abbaurate erheblich verringert, was auf die gesteigerte Hydrophobizität zurückgeführt wird.

Wegen der bevorzugten Anwendung der erfindungsgemäßen dreidimensionalen Gerüste für Gewebezüchtung, wurde die Zytotoxizität dieser Gerüste in Verbindung mit primären Epithelzellen und aus Fettgeweben gewonnenen Stammzellen (ASC) untersucht, wobei keine Zytotoxizität für Zellen in einem Medium festgestellt werden konnte, das zuvor mit einem Polymer 2 aufbereitet wurde, das einen besonders hohen Anteil an Polyphosphazenen aufweist. Weiterhin zeigten Vorstudien auch keine Zytotoxizität des Polymers 2 in einem Zellkulturmedium bei 37 °C während einer Zeitspanne von 42 Tagen, in denen bereits 33 % des Polymers abgebaut wurden, was auf die Nicht-Toxizität der Abbauprodukte oder deren Zwischenprodukte schließen lässt.

## Patentansprüche

1. Polymer für die Gewebezüchtung aus bioabbaubaren Polyphosphazenen mit photopolymerisierten Seitengruppen, **dadurch gekennzeichnet, dass** die Seitengruppen der Polyphosphazene ausschließlich aus Aminosäuren und/oder Aminosäurederivaten gebildet sind, wobei diese über die Aminogruppe der Aminosäure am Rückgrat des Polyphosphazens gebunden sind und an der Säuregruppe einen Abstandhalter aufweisen, dessen Kohlenstoffkette mit der Länge m = 0 bis m = 10 vorliegt, welcher am freien Ende eine Vinylgruppe aufweist, wobei die Polyphosphazene über die Vinylgruppen der Seitengruppen vernetzt sind.

2. Polymer nach Anspruch 1, **dadurch gekennzeichnet, dass** die photopolymerisierten Seitengruppen der Strukturformel 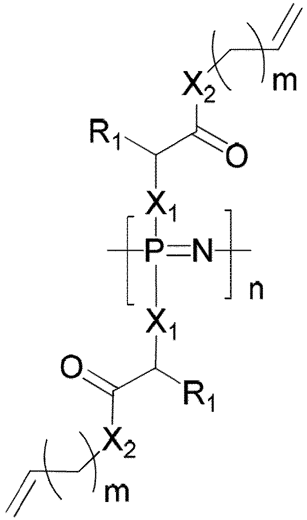 entsprechen, wobei X₁ für NH und X₂ für O, S oder NH steht und R₁ die Seitenkette von Alanyl, Valinyl, Leucinyl, Isoleucinyl, Prolinyl, Phenylalaninyl, Tryptophanyl, Methioninyl, Glycinyl, Serinyl, Threoninyl, Cysteinyl, Tyrosinyl, Asparaginyl, Glutainyl, Aspartoyl, Glutaoyl, Lysinyl, Argininyl oder Histidinyl ist und wobei gilt m = 0 bis 10 und n = 3 bis 1000.

3. Polymer nach einem der Ansprüche 1 bis 2, **dadurch gekennzeichnet, dass** die Seitengruppen aus Aminosäurevinylester oder Aminosäureallylester gebildet sind.

4. Polymer nach einem der Ansprüche 1 bis 3, **dadurch gekennzeichnet, dass** Vinylgruppen der Seitengruppen über zumindest zwei Thiolgruppen aufweisende Thiolverbindungen vernetzt sind.

5. Polymer nach Anspruch 4, **dadurch gekennzeichnet, dass** Vinylgruppen der Seitengruppen und Adipinsäuredivinylester über zumindest zwei Thiolgruppen aufweisende Thiolverbindungen vernetzt sind.

6. Polymer nach einem der Ansprüche 4 bis 5, **dadurch gekennzeichnet, dass** die Thiolverbindung drei Thiolgruppen aufweist und besonders bevorzugt als Trimethylolpropan-tris(3-mercaptopropionat) vorliegt.

7. Polymer nach einem der Ansprüche 1 bis 6, **dadurch gekennzeichnet, dass** es kein Hydrogel ist.

8. Polymer nach einem der Ansprüche 1 bis 7, **dadurch gekennzeichnet, dass** es formstabil ist.

9. Polymer nach einem der Ansprüche 1 bis 8, **dadurch gekennzeichnet, dass** es nicht quellend ist.

10. Polymer nach einem der Ansprüche 1 bis 9, **dadurch gekennzeichnet, dass** es porös ist.

11. Verfahren zur Herstellung eines porösen Polymers gemäß Anspruch 10, **dadurch gekennzeichnet, dass** in der Polymermatrix homogen verteilt auslaugbares Porogen, wie insbesondere Salz, eingeschlossen ist, welches nach Polymerisation ausgelaugt wird.

12. Verwendung eines Polymers nach einem der Ansprüche 1 bis 11, **dadurch gekennzeichnet, dass** aus dem Polymer ein dreidimensionales Gerüst für die Gewebezüchtung gebildet wurde, indem das Polymer in der gewünschten Form bzw. Gestalt photopolymerisiert bzw. photovernetzt wurde.

13. Verwendung eines Polymers nach Anspruch 12, **dadurch gekennzeichnet, dass** das photopolymerisierte bzw. photovernetzte Polymer starr und porös ist.

## Claims

1. A polymer for growing tissue from biodegradable polyphosphazenes having photopolymerised side groups, **characterised in that** the side groups of the polyphosphazenes are formed exclusively of amino acids and/or amino acid derivatives, wherein these are bound to the backbone of the polyphosphazene via the amino group of the amino acid and have a spacer at the acid group, the carbon chain of said having a vinyl group at its free end, wherein the polyphosphazenes are crosslinked via the vinyl groups.

2. The polymer of claim 1, **characterised in that** the photopolymerised side groups are of the structural formula 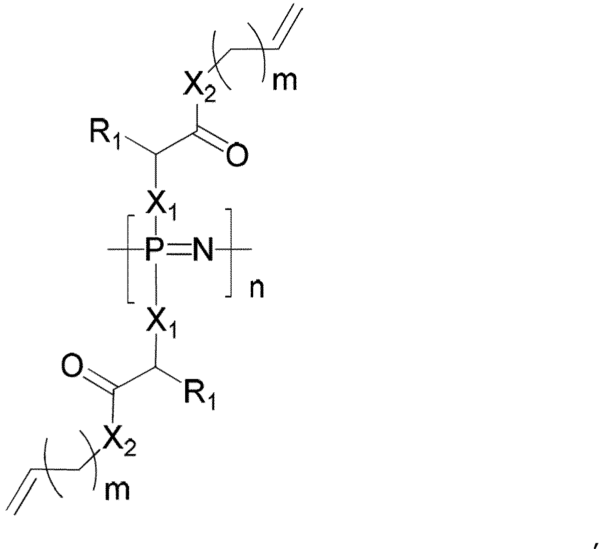 wherein X₁ represents NH and X₂ represents O, S or NH and R₁ is the side chain of alanyl, valinyl, leucinyl, isoleucinyl, prolinyl, phenylalaninyl, tryptophanyl, methioninyl, glycinyl, serinyl, threoninyl, cysteinyl, tyrosinyl, asparaginyl, glutainyl, aspartoyl, glutaoyl, lysinyl, argininyl or histidinyl and wherein m = 0 to 10 and n = 3 to 1000.

3. The polymer of any one of claims 1 to 2, **characterised in that** the side groups are formed of amino acid vinyl ester or amino acid allyl ester.

4. The polymer of any one of claims 1 to 3, **characterised in that** vinyl groups of the side groups are crosslinked via at least two thiol compounds having thiol groups.

5. The polymer of claim 4, **characterised in that** vinyl groups of the side groups and adipic acid divinyl esters are crosslinked via at least two thiol compounds having thiol groups.

6. The polymer of any one of claims 4 and 5, **characterised in that** the thiol compound has three thiol groups and particularly preferably is present as a trimethylolpropane-tris(3-mercaptopropionate).

7. The polymer of any one of claims 1 to 6, **characterised in that** it is not a hydrogel.

8. The polymer of any one of claims 1 to 7, **characterised in that** it is dimensionally stable.

9. The polymer of any one of claims 1 to 8, **characterised in that** it is non-swelling.

10. The polymer of any one of claims 1 to 9, **characterised in that** it is porous.

11. A method for preparing a porous polymer of claim 10, **characterised in that** leachable porogen, such as, in particular, salt, which is leached following polymerisation, is homogenously distributed within the polymer matrix.

12. Use of the polymer of any one of claims 1 to 11, **characterised in that** a three-dimensional frame for growing tissue has been formed from the polymer by photopolymerising or photocrosslinking the polymer in the form or shape desired.

13. Use of the polymer of claim 12, **characterised in that** the photopolymerised or photocrosslinked polymer is rigid and porous.

## Revendications

1. Polymère pour la culture de tissus à partir de polyphosphazènes biodégradables ayant des groupes latéraux photopolymérisés, **caractérisé en ce que** les groupes latéraux des polyphosphazènes sont formés exclusivement d'acides aminés et/ou de dérivés d'acides aminés, ceux-ci étant liés au squelette du polyphosphazène par l'intermédiaire du groupe amino de l'acide aminé et présentant, sur le groupe acide,un espaceur dont la chaîne carbonée est présente avec une longueur de m = 0 à m = 10 et qui présente un groupe vinyle à l'extrémité libre, les polyphosphazènes étant réticulés par l'intermédiaire des groupes vinyle des groupes latéraux.

2. Polymère selon la revendication 1, **caractérisé en ce que** les groupes latéraux photopolymérisés correspondent à la formule suivante 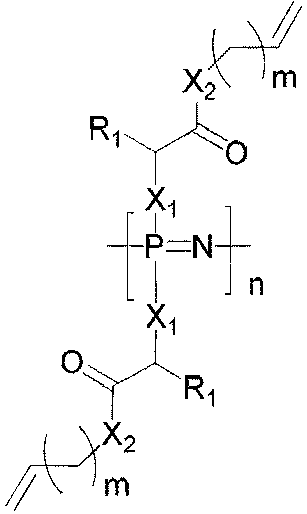 où X₁ est NH et X₂ est O, S ou NH et R₁ est la chaîne latérale d'alanyle, de valinyle, de leucinyle, d'isoleucinyle, de prolinyle, de phénylalaninyle, de tryptophanyle, de méthioninyle, de glycinyle, de sérinyle, de thréoninyle, de cystéinyle, de tyrosinyle, d'asparaginyle, de glutainyle, d'aspartoyle, de glutaoyle, de lysinyle, d'argininyle ou d'histidinyle, et où m = 0 à 10 et n = 3 à 1000.

3. Polymère selon l'une des revendications 1 à 2, **caractérisé en ce que** les groupes latéraux sont formés à partir d'un ester vinylique d'acide aminé ou d'un ester allylique d'acide aminé.

4. Polymère selon l'une des revendications 1 à 3, **caractérisé en ce que** les groupes vinyle des groupes latéraux sont réticulés à l'intermédiaire de composés thiol qui présentent au moins deux groupes thiol.

5. Polymère selon la revendication 4, **caractérisé en ce que** les groupes vinyle des groupes latéraux et les ester vinylique d'acide adipique sont réticulés à l'intermédiaire de composés thiol qui présentent au moins deux groupes thiol.

6. Polymère selon l'une des revendications 4 à 5, **caractérisé en ce que** le composé thiol présente trois groupes thiol et est présent, de manière particulièrement préférée, sous forme de triméthylolpropane-tris(3-mercaptopropionate) .

7. Polymère selon l'une des revendications 1 à 6, **caractérisé en ce qu'**il n'est pas un hydrogel.

8. Polymère selon l'une des revendications 1 à 7, **caractérisé en ce qu'**il résiste à la déformation.

9. Polymère selon l'une des revendications 1 à 8, **caractérisé en ce qu'**il ne gonfle pas.

10. Polymère selon l'une des revendications 1 à 9, **caractérisé en ce qu'**il est poreux.

11. Procédé de préparation d'un polymère poreux selon la revendication 10, **caractérisé en ce qu'**un porogène lessivable, tel que notamment un sel, est enfermé dans la matrice polymère et y est réparti de manière homogène, ledit porogène étant lessivé après la polymérisation.

12. Utilisation d'un polymère selon l'une des revendications 1 à 11, **caractérisée en ce qu'**un squelette tridimensionnel pour la culture de tissus a été formé à partir dudit polymère par photopolymérisation ou photoréticulation du polymère sous la forme souhaitée.

13. Utilisation d'un polymère selon la revendication 12, **caractérisée en ce que** le polymère photopolymérisé ou photoréticulé est rigide et poreux.
